# EUROPEAN PATENT SPECIFICATION

(11) **EP 0 395 103 B1**
(45) Date of publication and mention of the grant of the patent: **23.06.1993**
(21) Application number: 90108109.1
(22) Date of filing: 27.04.1990
(51) Int. Cl.: C07C 309/81, C07C 303/22

(54) **Process for preparing perfluoroalkenyl sulfonyl fluorides**
Verfahren zur Herstellung von Perfluoralkenylsulfonylfluoriden
Procédé de préparation de fluorures sulfonyliques de perfluoro alcènes

(30) Priority: 28.04.1989 IT 2033889
(43) Date of publication of application: 31.10.1990
(73) Proprietor: AUSIMONT S.p.A., I-20121 Milano (IT)
(72) Inventor: Navarrini, Walter, Dr., I-20010 Boffalora Ticino, Milan (IT); Desmarteau, Darryl D., Dr., 1007 Berkely Drive, 29631 S. Carolina (US)
(74) Representative: Barz, Peter, Dr.

(56) References cited:
- AT-B- 0 006 633
- DE-A- 3 047 439
- US-A- 3 041 317

## Description

The present invention relates to a process for the preparation of perfluoroalkenyl sulfonyl fluorides, in particular of perfluorovinyl sulfonyl fluoride.

As it is known, the perfluoroalkenyl sulfonyl fluorides are monomers for the preparation of fluorinated polymers containing sulfonyl functions, said polymers being suitable for many applications.

In US-A-3,041 317 a synthesis of perfluoroalkenyl sulfonyl fluorides of formula

R_{f}-CF=CF-SO₂F

is reported, wherein R_{f} is F or a perfluoroalkyl radical or an omega-hydroperfluoroalkyl radical.

The above synthesis uses as the starting materials 2-hydroperfluoroalkyl-sulfonyl fluorides of general formula

R_{f}-CF₂-CFH-SO₂F,

which are dehydrofluorinated in order to yield perfluoroalkenyl sulfonyl fluorides. The reaction is carried out inside a plant wherein a stream of reactants is passed under reduced pressure onto a catalyst composed of chromium oxide supported on KCl, at temperatures of from 450 to 630°C.

In particular, in case of perfluorovinyl sulfonyl fluoride, the reaction is carried out at temperatures of from 508 to 517°C, with a conversion of 51% and a yield of 61%, based on the starting material converted per each single run.

R.E. Banks in J. Chem. Soc. (C), 1966, reports a synthesis of perfluorovinyl sulfonyl fluoride in which the same catalyst and the same operating conditions as in the above US-A-3,041,317 are used, the only difference being that the catalyst bed is preheated at 510°C before the synthesis is carried out.

In the paper by R.E. Banks, the general reactivity of said molecule is also described.

In both of the above processes, the starting materials are 2-hydroperfluoroalkyl-sulfonyl fluorides of general formula

R_{f}-CFH-CF₂-SO₂F

which may be synthesized by means of the controlled hydrolysis of the corresponding sultones of formula 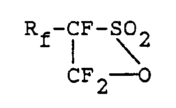
as described by D.C. England in J. Am. Chem. Soc. 1960, 82, 6181.

It has now surprisingly been found that the synthesis of 2-perfluoroalkenyl sulfonyl fluorides can be carried out continuously and under atmospheric pressure by using an inexpensive and readily available reactant and operating at process temperatures which are considerably lower than the temperatures used in the known processes.

Furthermore, when the reactants and the process conditions according to the present invention are used, a simplified synthesis is accomplished, which requries a lower number of intermediate steps. For example, the hydrolysis required by the known processes is no longer necessary in the process according to the present invention.

In particular, according to the present invention, a process for preparing perfluoroalkenyl sulfonyl fluorides of general formula (I)

R_{f}-CF=CF-SO₂F (I)

wherein:
- R_{f}: is F or a perfluoroalkyl radical of from 1 to 9 carbon atoms;

is provided, which comprises the contacting of a starting material, i.e., a perfluoroalkyl-(sulfonyl fluoride) monofluoroacetyl-fluoride of general formula (II)

R_{f}-CF₂-CF(COF)-SO₂F (II)

wherein R_{f} is as defined above, with a reactant selected from carbonates of metals belonging to Groups IA, IIA or IIB of the Periodic Table of Elements or mixtures of said carbonates at a temperature of from about 150 to about 400°C, preferably of from about 200 to about 350°C, whereafter a compound having the above formula (I) may be recovered from the effluent of the reaction.

The process according to the present invention is advantageously carried out in continuous manner, preferably under atmospheric pressure, by causing a gaseous stream of a perfluoroalkyl-(sulfonyl fluoride) monofluoroacetyl-fluoride used as starting material of formula (II) to flow, in the form of a vapour in an inert carrier, preferably nitrogen, over or through a bed of the reactant (carbonate) present inside a suitable reactor. For example, as the reactor a glass column or a steel column may be used, which preferably contains a bed of the carbonate reactant in a very finely subdivided form. In the reactor also a packing can be used, e.g., small-sized pieces of crushed glass.

The reactant used according to the present invention preferably is selected from Na₂CO₃, CaCO₃, MgCO₃, ZnCO₃, and mixtures thereof.

The reaction on which the process according to the present invention is based can be illustrated as follows for the case when CaCO₃ is used as the carbonate reactant.

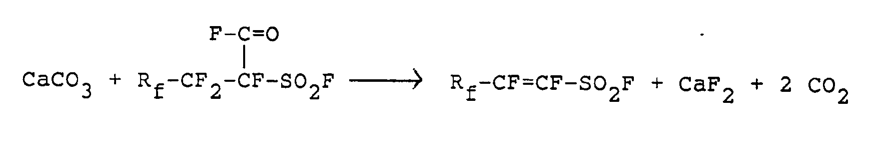

The amount of reactant used generally is at least equal to the stoichiometric amount required for the above reaction. Preferably an excess over the stiochiometric amount is used, also depending on the degree of comminution of the reactive bed.

The process generally is carried out under anhydrous conditions, with the carrier nitrogen and the reactant bed as well as the reactor packing, if used, being previously thoroughly dried, e.g., by means of heating at about 300°C for several hours. Nitrogen may then be passed through a charging trap containing a starting material (II), wherein said nitrogen is saturated with the vapours of said starting material (II), and may then be passed through the reactor maintained at the reaction temperature, at a flow rate such as to obtain a residence time of the starting material in the reactor of from about 3 to about 15 seconds.

The gas stream leaving the reactor may be passed through a condenser maintained at a sufficiently low temperature to condense all of the reaction products, but not nitrogen, e.g., at a temperature of from -98°C to -196°C.

The condensed crude reaction product may be distilled under reduced pressure and the vapours from the distillation may be condensed through a plurality of steps in order to first recover the desired perfluoroalkenyl sulfonyl fluoride product (I) and then SO₂ as reaction byproduct. Other byproducts from the process, such as CO₂ and SiF₄, as well as decomposition products such as, e.g., CF₃COF and C₂F₄, may be removed by applying a dynamic vacuum during the distillation.

The conversion of the starting material generally is complete, with yields of the desired product (I), defined as the ratio of the produced mols of product (I) to the reacted mols of starting material (II), within the range of from 30 to 50%.

The starting materials (II) used in the process according to the present invention may be synthesized from the corrresponding perfluoroalkylene sultones of formula (III): 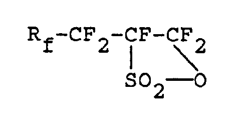
by means of isomerization in the presence of catalytic amounts of KF or of trialkylamines, with quantitative yields being obtained, as described by D.C. England in J. Chem. Soc. 1960, 82, 6181.

The process according to the present invention is particularly useful for the preparation of compounds of formula (I) wherein R_{f} is either F or a perfluoroalkyl group of from 1 to 3 carbon atoms. Most desirable results may be obtained in the preparation of trifluorovinyl sulfonyl fluoride, perfluoropropenyl sulfonyl fluoride and of perfluorobutenyl sulfonyl fluoride.

In particular, for the preparation of trifluorovinyl sulfonyl fluoride

CF₂=CF-SO₂F

2,3,3,3-tetrafluoro-2-fluorosulfonyl-propionyl fluoride of formula

CF₃-CF(COF)-SO₂F

is used. The latter compound may easily be synthesized by isomerization of perfluoropropylene sultone 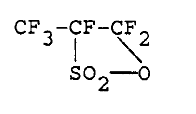
according to the above method of D.C. England.

The following examples are to illustrate preferred embodiments of the present invention.

### Example 1

In a continuously working plant 1.2 g (5.2 mmol) of

CF₃-CF(COF)-SO₂F

[2,3,3,3-tetrafluoro-2-(fluorosulfonyl)-propionyl fluoride] were placed into a charging trap of "U"-shape.

The charging trap subsequently was connected to the continuously working plant by means of valves.

Previously thoroughly dried nitrogen was passed, at a flow rate of 300 cm³/minute, through said charging trap, which was kept at a constant temperature of -35°C throughout the entire experiment.

Nitrogen leaving the charging trap, saturated with the vapours of the starting material, was passed through the reactor which was maintained at 300°C.

Over about 1.5 hours, all of the starting material was conveyed through the reactor (a glass column of 1.5 cm of diameter, filled with 40 g of sodium carbonate previously thoroughly dried at 300°C and packed with 40 g of small pieces of crushed glass).

The gases leaving the reactor were passed through two traps kept at -196°C, so as to condense all of the reaction products. The nitrogen carrier was discharged.

The crude reaction product contained inside both of said collecting traps was distilled under a pressure of 10⁻³ torr. The vapours coming from the distillation kettle were passed through cold traps maintained at temperatures of -80°C and -110°C, respectively.

Inside the trap at -110°C, 0.4 mmol of SO₂ condensed; inside the trap at -80°C, 1.6 mmol of trifluorovinyl sulfonyl fluoride

CF₂=CF-SO₂F

were obtained. CO₂, SiF₄ and the decomposition products, such as, e.g., CF₃COF and C₂F₄ were removed by applying a dynamic vacuum during the distillation.

The conversion of the starting material was complete.

The yield, defined as the ratio of mols of desired product (CF₂=CF-SO₂F) to the reacted mols of starting material, was 30%.

### Example 2

When following the procedure of Example 1, using a reactor temperature of 250°C, a flow rate of carrier nitrogen of 400 cm³/minute and a temperature of the charging trap of -10°C, the reaction yield of trifluorovinyl sulfonyl fluoride, as defined in Example 1, was 40%.

### Example 3

By following the procedure of Example 1, but using perfluoro-2-(fluorosulfonyl)-butanoyl fluoride as the starting material, perfluoropropenyl-sulfonyl fluoride was prepared.

### Example 4

When following the procedure of Example 1, using a reactant bed of CaCO₃, the yield of trifluorovinyl sulfonyl fluoride, as defined in Example 1, was 18%.

## Claims

1. Process for preparing perfluoroalkenyl sulfonyl fluorides of general formula (I)
R_{f}-CF=CF-SO₂F (I)
wherein:
R_{f} is selected from F and perfluoroalkyl radicals of from 1 to 9 carbon atoms,
comprising the contacting of a perfluoroalkyl-(sulfonyl fluoride) monofluoroacetyl-fluoride of general formula (II)
R_{f}-CF₂-CF(COF)-SO₂F (II)
wherein R_{f} is as defined above, with a reactant selected from carbonates of metals belonging to Groups IA, IIA and IIB of the Periodic Table of Elements or mixtures thereof at a temperature of from about 150 to about 400°C.

2. Process according to claim 1 wherein R_{f} is F or a perfluoroalkyl group containing from 1 to 3 carbon atoms.

3. Process according to any one of claims 1 and 2 wherein said temperature ranges from about 200 to about 350°C.

4. Process according to any one of claims 1 to 3 wherein said reactant is selected from Na₂CO₃, CaCO₃, MgCO₃, ZnCO₃ and mixtures thereof.

5. Process according to any one of claims 1 to 4, carried out continuously and under atmospheric pressure.

6. Process according to any one of claims 1 to 5, wherein the step of contacting is accomplished by causing a gaseous stream comprising an inert carrier saturated with the vapours of the compound of general formula (II) to flow over or through a bed of said reactant.

7. Process according to claim 6, wherein said inert carrier is nitrogen.

## Patentansprüche

1. Verfahren zur Herstellung von Perfluoralkenylsulfonylfluoriden der allgemeinen Formel (I)
R_{f}-CF=CF-SO₂F (I)
worin R_{f} ausgewählt ist aus F und Perfluoralkylresten mit von 1 bis 9 Kohlenstoffatomen,
umfassend das Inkontaktbringen eines Perfluoralkyl-(sulfonylfluorid)-monofluoracetylfluorids der allgemeinen Formel (II)
R_{f}CF₂-CF(COF)-SO₂F (II)
worin R_{f} wie oben definiert ist, mit einem Reaktanten, der ausgewählt ist aus Carbonaten von Metallen der Gruppen IA, IIA und IIB des Periodensystems oder Gemischen davon, bei einer Temperatur von etwa 150 bis etwa 400°C.

2. Verfahren nach Anspruch 1, worin R_{f} F oder eine 1 bis 3 Kohlenstoffatome enthaltende Perfluoralkylgruppe darstellt.

3. Verfahren nach irgendeinem der Ansprüche 1 und 2, worin die Temperatur im Bereich von etwa 200 bis etwa 350°C liegt.

4. Verfahren nach irgendeinem der Ansprüche 1 bis 3, worin der Reaktant ausgewählt ist aus Na₂CO₃, CaCO₃, MgCO₃, ZnCO₃ und Gemischen davon.

5. Verfahren nach irgendeinem der Ansprüche 1 bis 4, das kontinuierlich und unter Atmosphärendruck durchgeführt wird.

6. Verfahren nach irgendeinem der Ansprüche 1 bis 5, worin das Inkontaktbringen vorgenommen wird, indem man einen mit den Dämpfen der Verbindung der allgemeinen Formel (II) gesättigten, einen inerten Träger enthaltenden Gasstrom über oder durch ein Bett des Reaktanten strömen läßt.

7. Verfahren nach Anspruch 6, worin der inerte Träger Stickstoff ist.

## Revendications

1. Procédé de préparation de fluorures de perfluoro-alkényl-sulfonyle de formule générale (I):
R_{f}-CF=CF-SO₂F (I)
dans laquelle:
R_{f} est choisi dans le groupe comprenant F et les radicaux perfluoroalkyles comprenant 1 à 9 atomes de carbone,
comprenant le contact d'un fluorure monofluoroacétylique du fluorure de sulfonyle perfluoroalkylé de formule générale (II):
R_{f}-CF₂-CF(COF)-SO₂F (II)
dans laquelle:
R_{f} est tel que défini ci-dessus,
avec un réactif choisi parmi les carbonates des métaux appartenant aux groupes IA, IIA ou IIB de la Classification Périodique des Eléments ou de leurs mélanges, à une température comprise entre environ 150 et environ 400°C.

2. Procédé selon la revendication 1, dans lequel R_{f} est F ou un radical perfluoroalkyle contenant 1 à 3 atomes de carbone.

3. Procédé selon l'une des revendications 1 ou 2, dans lequel ledit domaine de température est compris entre 200°C environ et 350°C environ.

4. Procédé selon l'une quelconque des revendications 1 à 3, caractérisé en ce que le réactif est choisi dans le groupe comprenant Na₂CO₃, CaCO₃, MgCO₃, ZnCO₃ et leurs mélanges.

5. Procédé selon l'une quelconque des revendications 1 à 4, caractérisé en ce qu'il est effectué en continu et à pression atmosphérique.

6. Procédé selon l'une quelconque des revendications 1 à 5, caractérisé en ce que l'étape de contact est réalisée par passage d'un courant gazeux comprenant un support inerte saturé par des vapeurs du composé de formule générale (II) sur ou au travers d'un lit dudit réactif.

7. Procédé selon la revendication 6, caractérisé en ce que le support inerte consiste en azote.
